# EUROPEAN PATENT SPECIFICATION

(11) **EP 1 071 768 B1**
(45) Date of publication and mention of the grant of the patent: **29.12.2004**
(21) Application number: 99916683.8
(22) Date of filing: 16.04.1999
(51) Int. Cl.: C12N 15/12, A61K 48/00, C12N 5/10

(54) **DELIVERY OF THERAPEUTIC PROTEINS VIA IMPLANTATION OF GENETICALLY MODIFIED CELLS IN THE OMENTUM**
VERABREICHUNG DER THERAPEUTISCHEN PROTEINE DURCH IMPLANTATION DER GENETISCHEN MODIFIZIERTEN ZELLEN IN DAS OMENTUM
APPORT DE PROTEINES THERAPEUTIQUES PAR IMPLANTATION DE CELLULES GENETIQUEMENT MODIFIEES DANS L'EPIPLOON

(30) Priority: 24.04.1998 US 82982 P
(43) Date of publication of application: 31.01.2001
(73) Proprietor: Transkaryotic Therapies, Inc., Cambridge, MA 02139 (US)
(72) Inventor: LAMSA, Justin, Chase, Westminster, MA 01473 (US); TRECO, Douglas, A., Arlington, MA 02174 (US)
(74) Representative: Grund, Martin, Dr.
(86) International application number: PCT/US1999/008266
(87) International publication number: WO 1999/055866

(56) References cited:
- WO-A-90/12604
- WO-A-93/09222
- WO-A-95/25547
- WO-A-98/15615
- MOULLIER P ET AL: "Long-term delivery of a lysosomal enzyme by genetically modified fibroblasts in dogs." NATURE MEDICINE, (1995 APR) 1 (4) 353-7. , XP002115392
- DWARKI V J ET AL: "GENE THERAPY FOR HEMOPHILIA A: PRODUCTION OF THERAPEUTIC LEVELS OF HUMAN FACTOR VIII IN VIVO IN MICE" PROCEEDINGS OF THE NATIONAL ACADEMY OF SCIENCES OF USA, vol. 92, no. 4, 14 February 1995 (1995-02-14), pages 1023-1027, XP000569659 ISSN: 0027-8424
- CHUAH ET AL: "BONE MARROW STROMAL CELLS AS TARGETS FOR GENE THERAPY OF HEMOPHILIA A" HUMAN GENE THERAPY, vol. 9, no. 3, 10 February 1998 (1998-02-10), pages 353-365, XP002090778 ISSN: 1043-0342

## Description

The field of the invention is a method of delivering proteins, including therapeutic proteins such as clotting factors.

### Background of the Invention

Hemophilia A is caused by a deficiency in the blood clotting factor, Factor VIII, which is the final enzyme in the blood coagulation cascade. Its deficiency is characterized by bleeding in soft tissues, muscles and weight-bearing joints.

The treatment of Hemophilia A using gene therapy is especially attractive as the precise regulation of Factor VIII is not required, even a modest replacement is of significant benefit to patients, and the therapeutic range of Factor VIII levels is extremely wide.

A variety of gene therapy techniques have been used in animal models to study the long term delivery of Factor VIII. In one study, the administration of an adenovirus vector expressing a human Factor VIII (hFVIII) to a Factor VIII-deficient mouse resulted in the detection of hFVIII in the mouse plasma for more than five months following administration. In another report, an adenovirus vector expressing a hFVIII cDNA was administered by peripheral vein infusion to Factor VIII-deficient dogs. In these animals, the hemophilic phenotype was corrected and hFVIII was measured in quantities above the therapeutic level. Another group used an adenovirus system to insert an hFVIII construct into primary mouse fibroblasts *in vitro*. The mice in which these cells were implanted had a detectable systemic level of hFVIII for only a short time following intrasplenic implantation. In other studies, a retrovirus vector system was used to generate hFVIII-expressing human skin fibroblasts. These cells were implanted by the subcutaneous (SC) route into immune deficient mice. Although no human Factor VIII was detectable in plasma samples of the recipient mice, these investigators were able to demonstrate long-term persistence of the transplanted cells. Another study using a retrovirus system to introduce the hFVIII gene into primary human skin fibroblasts demonstrated that hFVIII-expressing fibroblasts embedded in collagen and implanted by the intraperitoneal (IP) route resulted in detectable hFVIII in plasma for at least seven days. These investigators also observed that hFVIII was delivered systemically more efficiently by cells implanted via the intraperitoneal (IP) versus the intramuscular (IM) route.

### Summary of the Invention

The invention is based on the discovery of a method which allows for the long-term systemic delivery of a protein, e.g., a therapeutic protein such as a clotting factor. Delivery of the latter is useful in the prevention or treatment of disorders associated with coagulation and thrombosis.

The present method of administering a protein includes the steps of identifying an animal (e.g., a mammal such as a human, a non-human primate, a dog, a cat, a cow, a pig, a goat, a sheep, a horse, a rabbit, a mouse, a rat, a guinea pig, or a hamster) in need of a particular therapeutic protein, and introducing into the omentum of that animal a cell that secretes the protein. The protein can be a clotting factor, such as any of the known clotting factors, e.g., Factor VIII or IX. The cell can be transfected (or infected, if a viral vector is used) with a sequence that encodes the therapeutic protein. In another embodiment, the cell expresses an endogenous gene as a result of a genetic manipulation, e.g., the cell is transfected or infected with a construct that, following homologous recombination with the genomic DNA of the cell, activates expression of a largely silent endogenous gene encoding the protein, for example by introducing an exogenous regulatory sequence that replaces or overrides the endogenous regulatory region of the endogenous gene.

The cell may be introduced into the omentum by surgical implantation, laparoscopy or direct injection, e.g., via CT-guided needle or ultrasound-guided needle. The cell may be, for example, an immortalized cell or a primary or secondary cell, e.g., a primary or secondary fibroblast.

As used herein, the term "clotting factor" means any factor that is involved in the blood clotting cascade, including the intrinsic, extrinsic and final common pathways. Specifically, it includes any factor associated with coagulation or thrombosis disorders. For example, Factor VIII deficiency is involved in Hemophilia A and Factor IX deficiency is involved in Hemophilia B. Both are contemplated by the present invention.

An "omentum" is any internal membranous structure containing a sheet of fat. Usually, it is a fold of peritoneum extending from stomach to adjacent abdominal organs. The greater omentum is attached to the bottom edge of the stomach and hangs down in front of the intestines, with the lower edge attached to the transverse colon. The lesser omentum is attached to the top edge of the stomach and extends to the undersurface of the liver.

By "infected cell" is meant a cell into which (or into an ancestor of which) a DNA molecule has been artificially introduced using a viral vector. Viruses known to be useful as vectors include adenovirus, adeno-associated virus, Herpes virus, mumps virus, poliovirus, lentivirus, retroviruses, Sindbis virus, and vaccinia viruses such as canary pox virus.

By "transfected cell" is meant a cell into which (or into an ancestor of which) a DNA molecule has been artificially introduced by a means other than using a viral vector.

### Brief Description of the Drawings

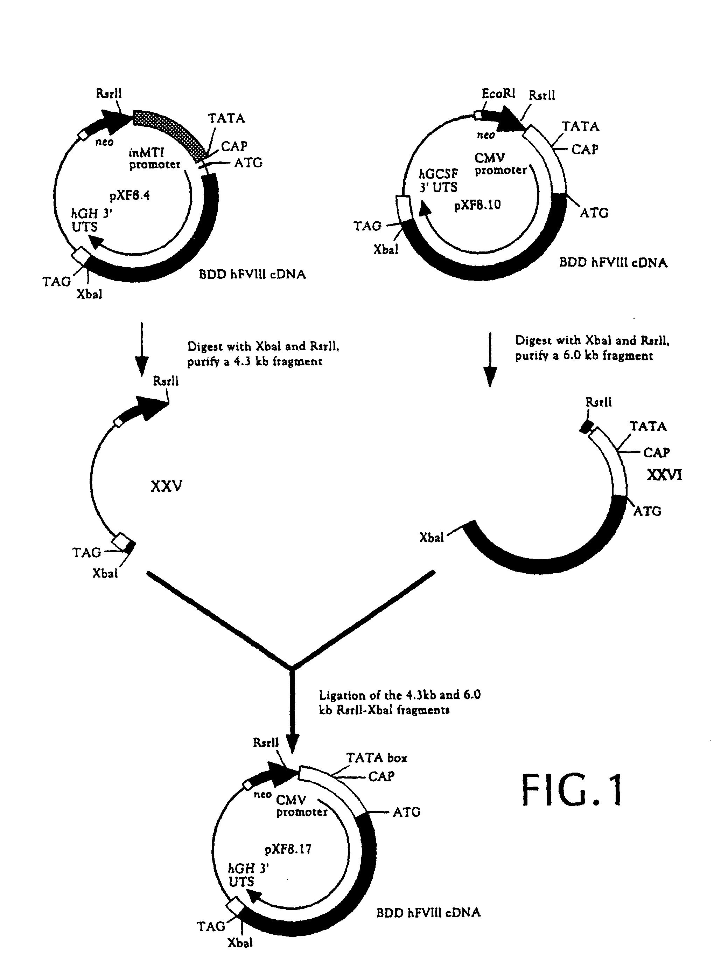

Fig. 1 is a schematic representation of the restriction map of plasmid pXF8.

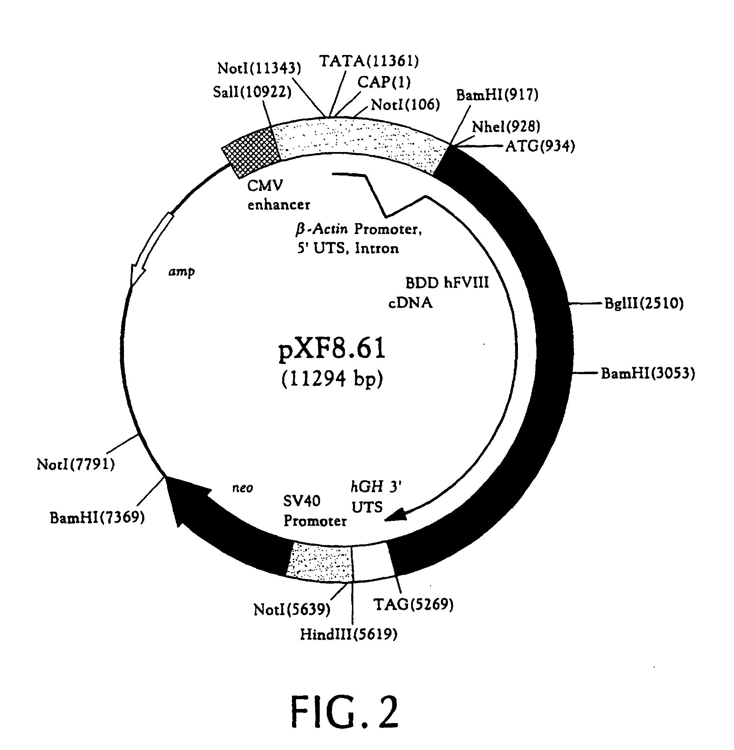

Fig. 2 is a schematic representation of the restriction map of plasmid pXF8.61.

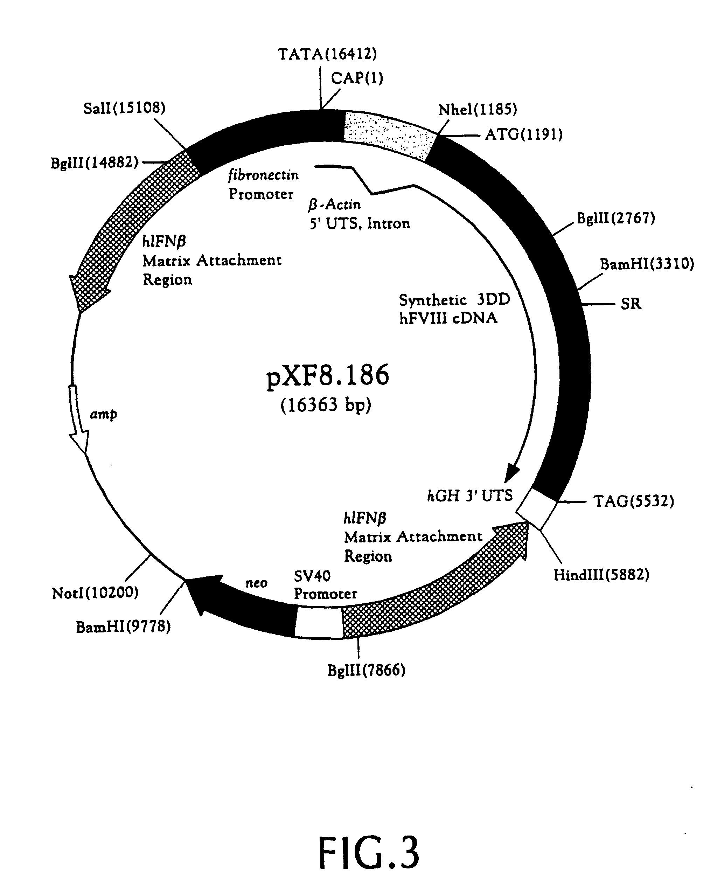

Fig. 3 is a schematic representation of the restriction plasmid map of plasmid pXF8.186.

### Detailed Description

The invention is based, in part, on the discovery of a method which allows for the long-term systemic delivery of any protein in an animal. The method includes introducing into the omentum of an animal (e.g., a mammal such as a human) cells expressing and secreting the protein at a therapeutically effective level. The invention thus includes use of such cells for the manufacture of a medicament for the treatment or prevention of a medical condition in a mammal by implantation of the cell in the omentum of the mammal, where the cell is genetically engineered to secrete a therapeutic protein useful in preventing or alleviating symptoms of the condition.

The invention includes a pharmaceutical composition for the treatment of a medical condition in a mammal. The pharmaceutical composition is adapted for introduction into the omentum of the mammal and includes (a) a cell genetically engineered to secrete a therapeutic protein and (b) a carrier.

The invention also includes a method of manufacturing a medicament which can be used to treat a medical condition in a mammal. The medicament is adapted for introduction into the omentum of the mammal. The method includes mixing (a) a cell which is genetically engineered to secrete a therapeutic protein and (b) a carrier.

The invention further includes a method of manufacturing a medicament for the treatment of a medical condition in a mammal, where the treatment comprises implantation of the medicament into the omentum of the mammal. The method includes mixing (a) a cell which is genetically engineered to secrete a therapeutic protein and (b) a carrier.

The cells employed by the present invention can be any vertebrate cell type (e.g., primary, secondary or immortalized cells) that is suitable for protein expression. Examples of immortalized human cells useful in the present method include, but are not limited to; a Bowes Melanoma cell (ATCC Accession No. CRL 9607), a Daudi.cell (ATCC Accession No. CCL 213), a HeLa cell and a derivative of a HeLa cell (ATCC Accession Nos. CCL 2, CCL2.1, and CCL 2.2), a HL-60 cell (ATCC Accession No. CCL 240), a HT1080 cell (ATCC Accession No. CCL 121), a Jurkat cell (ATCC Accession No. TIB 152), a KB carcinoma cell (ATCC Accession No. CCL 17), a K-562 leukemia cell (ATCC Accession No. CCL 243), a MCF-7 breast cancer cell (ATCC Accession No. BTH 22), a MOLT-4 cell (ATCC Accession No. 1582), a Namalwa cell (ATCC Accession No. CRL 1432), a Raji cell (ATCC Accession No. CCL 86), a RPMI 8226 cell (ATCC Accession No. CCL 155), a U-937 cell (ATCC Accession No. CRL 1593), WI-38VA13 sub line 2R4 cells (ATCC Accession No. CLL 75.1), a CCRF-CEM cell (ATCC Accession No. CCL 119) and a 2780AD ovarian carcinoma cell (Van Der Blick et al., Cancer Res. 48: 5927-5932, 1988), as well as heterohybridoma cells produced by fusion of human cells and cells of another species. In a preferred embodiment, the cell is a non-transformed cell. Examples of suitable mammalian cells include primary or secondary mammalian cells, e.g., a fibroblast, a hematopoietic stem cell, a myoblast, a keratinocyte, an epithelial cell, an endothelial cell, a glial cell, a neural cell, a cell comprising a formed element of the blood, a muscle cell and precursors of these somatic cells. In a most preferred embodiment, the cell is a secondary human fibroblast. For example, human skin fibroblasts can be used. Other suitable cells, such as other types of fibroblasts, keratinocytes, epithelial cells, endothelial cells, glial cells, neural cells, formed elements of the blood, muscle cells, hepatocytes and precursors of any of the foregoing, are known to those skilled in the art. As used herein, the term primary cells means (a) cells present in a suspension of cells isolated from a vertebrate tissue source (prior to their being plated, i.e., attached to a tissue culture substrate.such as a dish or flask); (b) cells present in an explant derived from tissue; (c) either (a) or (b) plated for the first time; or (d) cell suspensions derived from these plated cells. The terms secondary cell and cell strain refer to non-immortalized cells at all subsequent steps in culturing.

The cells are preferably compatible with the host animal, e.g., syngeneic or autologous, so that immune rejection of the cells is not an issue. However, if a suitable barrier device is employed, e.g., an encapsulation device such as a membrane polymer, any non-autologous cell (even xenogeneic cells) may be used. Alternatively, an immunosuppressive drug regimen can be administered to protect the cells from the host's immune system.

The cells can be prepared for implantation by any means known in the art. For example, confluent monolayers of clonal transfected or infected fibroblast strains may be digested with trypsin, and the resultant cell slurry centrifuged with PBS into a loose pellet for implantation.

The number of cells needed for a given dose depends on several factors, including the expression level of the protein, the size and condition of the host animal, and the limitations associated with the implantation procedure. Usually the number of cells implanted in an adult human or other similarly-sized animal is in the range of 1 X 10⁴ to 5 X 10¹⁰, and preferably 1 X 10⁸ to 1 X 10⁹. If desired, they may be implanted at multiple sites in the omentum, either at one time or over a period of months or years. The dosage may be repeated as needed.

The plasmid used to express the protein of interest can be introduced into a cell *ex vivo* by conventional transfection techniques. As used herein, the term "transfection" is intended to refer to a variety of art-recognized, non-viral techniques for introducing foreign nucleic acid (e.g., DNA) into a host cell, including calcium phosphate or calcium chloride co-precipitation, DEAE-dextran-mediated transfection, lipofection, biolistic transfer, naked DNA uptake, or electroporation. Suitable methods for transfecting host cells *in vitro* can be found in Sambrook et al., eds., Molecular Cloning: A Laboratory Manual (2nd ed., Cold Spring Harbor Laboratory Press, Cold Spring Harbor, NY, 1989) and other laboratory manuals.

For stable transfection or infection of mammalian cells, it is known that, depending upon the nature of the nucleic acid and the transfection or infection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same DNA molecule as that encoding the protein or can be introduced on a separate molecule. Cells stably transfected or infected with the introduced nucleic acid can be identified by drug selection (e.g., cells that have incorporated the selectable marker gene will survive, while the other cells die). Generally, a cell will be isolated and a clonal population produced and characterized prior to use in therapy.

Viral vectors such as retroviral, herpes, adenovirus, adenovirus-associated, mumps, canary-pox and poliovirus vectors can be used to introduce DNA into cells. Use of viral vectors is well known in the art: see, e.g., Robbins and Ghivizzani, "Viral Vectors for Gene Therapy" (1998) Pharmacol. Ther. 80:35-47; and Gunzburg and Salmons, "Virus Vector Design in Gene Therapy" (1995) Mol. Med. Today 1:410-7. Alternatively, non-viral nucleic acids can be used. Examples of non-viral mammalian expression plasmids include pCDM8 (Seed (1987) *Nature* 329:840) and pMT2PC (Kaufman et al. (1987) *EMBO J.* 6:187-195). When used in mammalian cells, the nucleic acid's expression control functions may be provided by viral or cellular regulatory elements. For example, commonly used promoters are derived from polyoma, Adenovirus 2, cytomegalovirus and Simian Virus 40. Non-viral promoters such as those derived from collagen and actin genes are described in U.S. Patent No. 5,641,670. Other viral and non-viral promoters are well known in the art. For other suitable expression systems, see chapters 16 and 17 of Sambrook et al. (supra).

The nucleic acid may have regulatory elements capable of directing expression of the nucleic acid preferentially in the particular cell type (e.g., fibroblasts), or the regulatory elements may be constitutively active.

The cell to be implanted may have been genetically engineered to express a gene encoding the desired protein. By "genetically engineered to express a gene" is meant that a DNA construct has been incorporated into the cell in a way that results in transcription and ultimately translation of the gene within the cell. The construct can be a viral vector, or can be a plasmid. In one embodiment, the DNA construct contains (a) a coding sequence encoding the protein and (b) one or more regulatory sequences (e.g., a promoter) which control expression of the coding sequence within the cell. Such a construct can be integrated into the genome of the cell (either randomly or at a target site by means of homologous recombination), or can exist episomally within the cell. Alternatively, instead of encoding the protein, the DNA construct may be designed to insert, by homologous recombination, an exogenous regulatory sequence into the genome of the cell at a position that permits the exogenous regulatory sequence to control expression of an endogenous gene encoding the protein. Such a construct would also contain one or more sequences homologous to a target site in the genome, and typically contains one or more selection marker genes as well. Using this construct, the regulatory region of the endogenous gene may be altered, replaced, or overridden such that the gene, which otherwise would be silent in the cell, is activated. This method is described in more detail in U.S. Patent No. 5,641,670.

The cells of the present invention can be introduced into the omentum by any means known in the art. The cells can be in a liquid suspension or imbedded in a solid composition such as a biocompatible polymer (e.g., collagen). Cells expressing the desired protein may be placed in a barrier device which allows the protein product to pass freely through the device while protecting and isolating the cells from the host's immune system. The use of barrier devices is well known to those in the art. The cells can be implanted into the omentum through a surgical procedure, or using standard laparoscopy or direct injection techniques.

The omentum implantation method of the present invention can be used to express any protein, such as growth hormone, insulin and α-galactosidase. Preferably the protein is a protein that can provide therapeutic benefit in a particular disease, e.g., Factor VIII in mammals with Hemophilia A, or Factor IX in those with Hemophilia B. The cells can express the full length protein or a truncated but biologically active form of the protein, e.g., the cells can express the full length Factor VIII protein or a B-domain deleted Factor VIII protein. Other types of proteins include erythropoietin; calcitonin; insulinotropin; insulin-like growth factors; parathyroid hormone; soluble parathyroid hormone receptor; interferons such as α interferon, β interferon, and γ interferon; nerve growth factors; glucagon; glucagon-like peptide 1; GLP-1 related peptide; interleukins; colony stimulating factors such as G-CSF and GM-CSF; immunoglobulins; catalytic antibodies; glucocerebrosidase; superoxide dismutase; tissue plasminogen activator; Factor IX; apolipoprotein E; apolipoprotein A-I; globins; soluble low density lipoprotein receptor; soluble IL-2 receptor; IL-2 antagonists; alpha-1 antitrypsin and immune response modifiers. If the protein is not naturally secreted, it can be modified by adding an appropriate secretion signal sequence.

The methods of the invention could also be used to deliver protein antigens which induce a protective immune response against viral, bacterial, or eukaryotic parasitic disease.

Any disease amenable to treatment with a systemically administered therapeutic protein or peptide can be treated by the present invention. Such diseases include those associated with a genetic defect such as any growth factor deficiency, blood coagulation disorder, immunodeficiency, hormone deficiency, hemoglobinopathy, storage disorder and coagulation factor deficiency.

The invention is further described in the following examples, which limit the scope of the invention described in the claims.

### Examples

### Example 1: Optimal site for implantation of clonal fibroblasts producing human Factor VIII

Stably transfected clonal fibroblasts were produced by transfection with B-domain deleted (BDD) hFVIII expression plasmids. Clonal cell strains were implanted into immunocompromised host mice in order to identify an optimal system for systemic delivery of BDD hFVIII.

### Isolation and Growth of Human Skin Fibroblasts

Stably transfected clonal human skin fibroblasts were produced by electroporation of cells with the BDD hFVIII expression plasmid pXFS.186.

### Implantation Methods

Confluent monolayers of clonal transfected fibroblast strains were digested with trypsin, and the resultant cell slurry centrifuged with PBS into a loose pellet for implantation. Female athymic nude mice, female *rag*-2 mice, or male *rag*-2 mice (Taconic Farms, Tarrytown, NY) were purchased at between 4 and 9 weeks of age and used for experiments when between 10 and 14 weeks of age. The mice were implanted with fibroblasts beneath the kidney capsule (SRC; 3 x 10⁶ cells/implant), within the mesentery surrounding the intestines (IP/M; 10 x 10⁶ cells/implant), in the lesser omental recess proximal to the stomach (IO; 1.25, 2.5, or 5 x 10⁶ cells/implant), into the muscle of the hind limb (IM; 3 x 10⁶ cells/implant), into the subcutaneous space (SC; 10 X 10⁶ cells/implant), or into the major mesenteric lymph node (IL; 3 x 10⁶, cells/implant).

### Intraperitoneal/Mesentery Implantation

Anesthetized animals were wiped with alcohol and prepped with Betadine. A small incision of 0.5 to 1 cm were made along the midline, and the abdominal viscera were exposed. The cells, contained in a plastic catheter, were injected within the abdomen upon the mesentery, in the region of the major mesenteric lymph node. The muscle layer was closed with a 4-0 absorbable suture attached to an atraumatic needle. The skin was closed using stainless steel staples.

### Intraperitoneal/Lymph Node Implantation

Anesthetized animals were wiped with alcohol and prepped with Betadine. A small incision of 0.5 to 1 cm was made along the midline and the abdominal viscera were exposed. The cells were injected into the mesenteric lymph node using a 23 gauge needle. The muscle layer was closed with a 4-0 absorbable suture attached to an atraumatic needle. The skin was closed using stainless steel staples.

### Intraomental Implantation

Anesthetized animals were placed in lateral recumbency and the left flank between the ribs and stifle wiped with an alcohol pad and prepped with Betadine. A small (0.5 cm to 1.0 cm) incision was made posterior to the ribs and ventral to the spine. The spleen was exposed and gently exteriorized. Cells were injected along the axis of the spleen within the thin membrane adjacent to the hilar surface of the spleen. The spleen was replaced in the abdominal cavity and the incision closed using stainless steel staples.

### Controls

Two sets of controls were established for use in the assay. Control 1 consisted of FACT (Factor VIII standards were prepared from pooled, citrated normal human plasma; FACT plasma, 1 U/mL, George King Biomedical, Overland Park, KS) plasma diluted 1:5, and frozen at -80° C until use. Control 1 was further diluted in either sample buffer or sample buffer with 20% NMP 1:5 for use in the assay. Control 2 consisted of full-length CHO cell-derived Factor VIII (FVIII) obtained from Genetics Institute (Cambridge, MA), diluted in sample buffer to an initial concentration of 1 µg/mL and stored frozen (-80° C). The 1 µg/mL stock solution was diluted to 40 ng/mL in sample buffer, and stored frozen (-80°C). Control 2 was further diluted for use in either sample buffer or sample buffer with 20% NMP 1:5.

### Elisa Assay

The lower detection limit of the hFVIII ELISA is approximately 3 mU/mL. The interassay variation is 8.6% while the interassay coefficient of variation is 4.9%.

### Immunoprecipitation and Western blotting

BDD hFVIII levels in selected samples were independently confirmed by immunoprecipitation and Western blot analysis. Selected samples from TKM 101 (implanted IO with RF 261 B2-106 cells) were collected as described above.

### Study animals

The following mouse strains were used:
(i) Athymic nude mouse: N-.NIH(S)-*nu*/*nu* nulliparous and non-pregnant female
(ii) *rag*-2 mouse: 129/SvEvTacfBr-[KO]*rag*-2 nulliparous and non-pregnant female
(iii) *rag*-2 mouse: 129/SvEvTacfBr-[KO]*rag*-2 male

### Cell Types

The clonal cell strains utilized in the studies are listed in Table 1.

**Table 1**

| **Stably Transfected Skin Fibroblasts Used in Animal Model Development Studies** | | | |
|---|---|---|---|
| **Cell Strain** | **Expression Construct** | **Expressed Protein** | **Example Studies** |
| RF 223.C10 | pXF8.17¹ | BDD hFVIII | TKM5 |
| RF 261 B2-106 | pXF8.61² | BDD hFVIII | TKM 92, |
| | | | 101, |
| | | | 108,111 |
| RF 288 B1-59 | pXF8.61² | BDD hFVIII | TKM 132 |
| RF 302 B2-382 | pXF8.186³ | BDD hFVIII | TKM 174 |
| RF 302 B2-383 | pXF8.186³ | BDD hFVIII | TKM 175 |
| RF 302 B2-373 | pXF8.186³ | BDD hFVIII | TKM 176 |
| HF 701-18 | pXF8.186³ | BDD hFVIII | TKW 17 |
| HF 701-27 | pXF8.186³ | BDD hFVIII | TKW 18 |

| | | | |
|---|---|---|---|
| ¹ CMV promoter construct; Fig. 1. | | | |
| ² β-actin promoter, CMV enhancer construct; Fig. 2. | | | |
| ³ Fibronectin promoter, β-interferon matrix attachment region; Fig. 3. | | | |

### Study Groups:

Table 2 summarizes the cells, animals, and implantation sites used in the animal model development studies.

**Table 2**

| **Preclinical Studies Performed in Immunocompromised Mice** | | | | | | |
|---|---|---|---|---|---|---|
| Study¹ | Cells | Cell Number | Implant | Mouse | Sex | N |
| TKM 5 | nTX | 10 x 10⁶ | IP | *nude* | F | 4 |
| TKM 5 | RF 223.C10 | 10 x 10⁶ | IP | *nude* | F | 10 |
| TKM 5 | RF 233.C10 | 3 x 10⁶ | SRC | *nude* | F | 10 |
| TKM 5 | RF 223.C10 | 10 x 10⁶ | SC | *nude* | F | 10 |
| TKM 5 | RF 223.C10 | 3 x 10⁶ | IM | *nude* | F | 10 |
| TKM 5 | RF 223.C10 | 3 x 10⁶ | IL | *nude* | F | 10 |
| TKM 93 | NA | NA | NA | *nude* | F | 5 |
| TKM 93 | RF 261 B2-106 | 5 x 10⁶ | IP/M | *nude* | F | 10 |
| TKM 100 | NA | NA | NA | *nude* | F | 10 |
| TKM 101 | RF 261 B2-106 | 10 x 10⁶ | IO | *nude* | F | 5 |
| TKW 108 | NA | NA | NA | *nude* | F | 5 |
| TKW 108 | RF 261 B2-106 | 5 x 10⁶ | IO | *nude* | F | 10 |
| TKW 108 | RF 261 B2-106 | 5 x 10⁶ | IO | *rag-2* | F | 8 |
| TKM 111 | NA | NA | NA | *nude* | F | 5 |
| TKM 111 | RF 261 B2-106 | 1.25 x 10⁶ | IO | *nude* | F | 10 |
| TKM 111 | RF 261 B2-106 | 2.5 x 10⁶ | IO | *nude* | F | 10 |
| TKM 111 | RF 261 B2-106 | 5 x 10⁶ | IO | *nude* | F | 10 |
| TKM 126 | NA | NA | NA | *nude* | F | 10 |
| TKM 132 | RF 288 B1-59 | 5 x 10⁶ | IO | *nude* | F | 10 |
| TKM 172 | NA | NA | NA | *nude* | F | 10 |
| TKM 174 | RF 302 B2-382 | 5 x 10⁶ | IO | *nude* | F | 10 |
| TKM 175 | RF 302 B2-383 | 5 x 10⁶ | IO | *nude* | F | 10 |
| TKM 176 | RF 302 B2-373 | 5 x 10⁶ | IO | *nude* | F | 10 |
| TKW 16 | NA | NA | NA | *rag-2* | M | 10 |
| TKW 17 | HF 701-18 | 5 x 10⁶ | IO | *rag-2* | M | 10 |
| TKW 18 | HF 701-27 | 5 x 10⁶ | IO | *rag-2* | M | 10 |

| | | | | | | |
|---|---|---|---|---|---|---|
| ¹ Studies sharing a common control (untreated or saline implanted) are enclosed within the same box in Table 2. In all studies control animals were from the same lot of mice and shared a common birth date with the experimental group. Thus, the control mice may have been used as control for multiple experiments; untransfected rabbit fibroblasts were used as a control in study TKM 5. | | | | | | |
| NA: not applicable; control animals either untreated or saline implanted. | | | | | | |
| IO: Intraomentum | | | | | | |

### Observation Points

Blood was collected via the retro-orbital sinus at regular intervals. Collections were weekly for up to 6 weeks after cell implantation, approximately every other week from 6 to 12 weeks after implantation, and monthly thereafter.

### Identification of a Suitable Implant Site for Long-Term Systemic Delivery of BDD hFVIII

Factors that might influence the effectiveness of a cell-based gene therapy system include the choice of expression system, animal model, and site of implantation. Expression systems that are potentially useful in clinical development were identified by implantation of stably transfected cells generated from different expression constructs into immunocompromised mice and screening for long-term BDD hFVIII delivery. The immunocompromised mouse system was chosen since it allows the implantation of xenogeneic fibroblasts without active rejection. The expression of hFVIII in fibroblasts alone is not sufficient for the consistent elevation of hFVIII in plasma; the site of implantation has now been shown to play a key role in the bioavailability of the protein. Following SC or IM injection of hFVIII in mice, the protein could not be detected in plasma, although uptake into the blood from an IP injection was observed. In another study, a single hemophilia A patient was injected with purified hFVIII SC, IP, or IV. The study showed that only direct intravascular (IV) injection resulted in detectable circulatory hFVIII; SC and IP injections were ineffective.

A series of studies designed to identify the most suitable implantation site for long-term systemic delivery of BDD hFVIII was performed. Initial studies employed stably transfected clonal rabbit skin fibroblasts (RF 233.C10) expressing BDD hFVIII (see Table 1). Experiment TKM 5 (see Table 2) was undertaken to evaluate BDD hFVIII delivery by transfected fibroblasts implanted in SC, IM, IL, SRC, and IP sites. The results of this study are summarized in Table 3.

**Table 3**

| **Evaluation of Implantation Sites: BDD hFVIII Delivery by Transfected Clonal Rabbit Skin Fibroblasts (TKM 5)** | | | | |
|---|---|---|---|---|
| Implant Site | No. Animals | Plasma hFVIII (mU/mL) | | RF223.C 10 Cells |
| | | Day 1 | Day 28* | |
| Subcutaneous (SC) | 10 | 0.0 ± 0.0 | 0.0 ± 0.0 | 10 x 10⁶ |
| Intramuscular (IM) | 9 | 2.6 ± 7.6 | 0.3 ± 0.9 | 3 x 10⁶ |
| Mesenteric Lymph node (IL) | 10 | 29.5 ± 21.3 | 0.0 ± 0.0 | 3 x 10⁶ |
| Subrenal capsular (SRC) | 10 | 62.1 ± 35.2 | 3.4 ± 9.2 | 3 x 10⁶ |
| Intraperitoneal (IP) | 10 | 199.3 ± 118.1 | 11.2 ± 35.4 | 10 x 10⁶ |
| Control: 10 x 10⁶ non-transfected rabbit fibroblasts (IP) | 4 | 0.0 ± 0.0 | 0.0 ± 0.0 | N/A |

| | | | | |
|---|---|---|---|---|
| * Plasma hFVIII not detected in any group after day 28 (study continued to day 114) | | | | |

Only a single animal in each of the SRC and IP groups exhibited expression after day 1 in this study. Correcting for the number of cells administered, it appears that systemic availability of BDD hFVIII was similar from the SRC and IP sites, and that these were preferred sites for BDD hFVIII delivery as compared to the SC, IM, and IL sites. In a subsequent experiment, the BDD hFVIII-expressing rabbit fibroblast strain RF 261 B2-106 was implanted IP/M into nude mice (experiment TKM 93; see Tables 1 and 2). The results demonstrated that transfected clonal rabbit skin fibroblasts expressing BDD hFVIII implanted in this manner did not result in long-term systemic delivery of BDD hFVIII, although the duration of detectable expression (3 weeks) was higher than seen in previous studies.

To further improve on the IP site for use in gene therapy, the omental recess was evaluated as a candidate site for implantation of stably transfected clonal fibroblasts expressing BDD hFVIII. To test the IO route for BDD hFVIII delivery, 5 x 10⁶ RF 261 B2-106 cells (the same strain used in experiment TKM 93) were implanted IO into ten nude mice, with an additional five mice serving as controls (TKM 108). Control animals remained at or near the detection limit of the assay for the duration of the experiment (Table 4). In contrast, BDD hFVIII plasma levels in treated animals remained elevated for 365 days (Table 5). In order to confirm that hFVIII antigen detectable by the ELISA assay corresponded to a plasma protein of the size predicted for BDD hFVIII, Western blot analysis was performed on selected samples from mice implanted IO with
RF 261 B2-106 cells. Fresh plasma samples drawn from five mice of experiment TKM 101 (see Table 2) were immunoprecipitated using an anti-hFVIII antibody and subjected to SDS-PAGE and Western blotting. Western blot analysis of samples obtained 91 days after implantation showed that plasma from mice implanted with BDD hFIII expressing cells have a clear anti-hFVIII reactive band at a size predicted for the single chain form of BDD hFVIII (174 kD). This band is absent from plasma obtained from untreated nude mice.

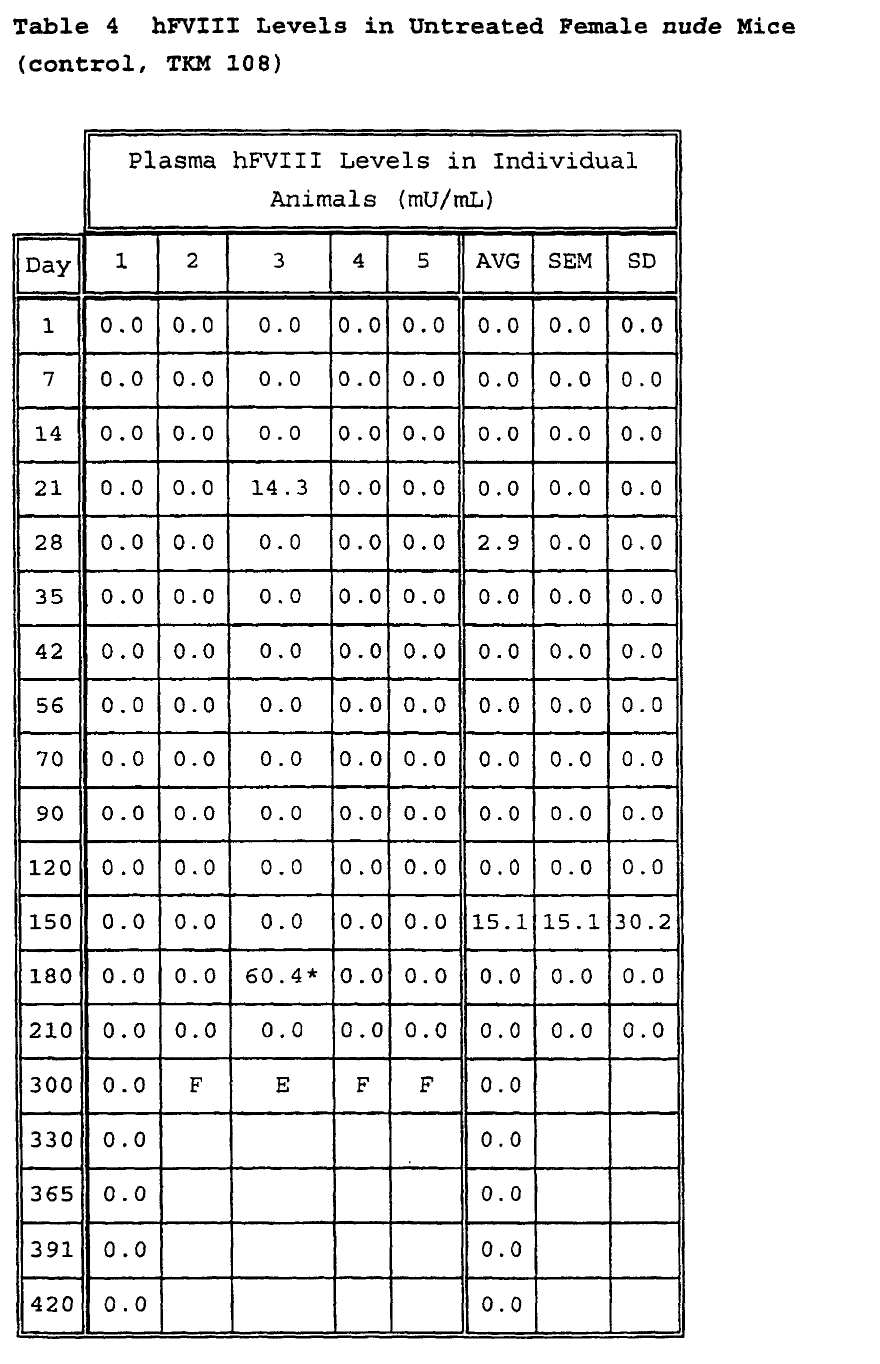

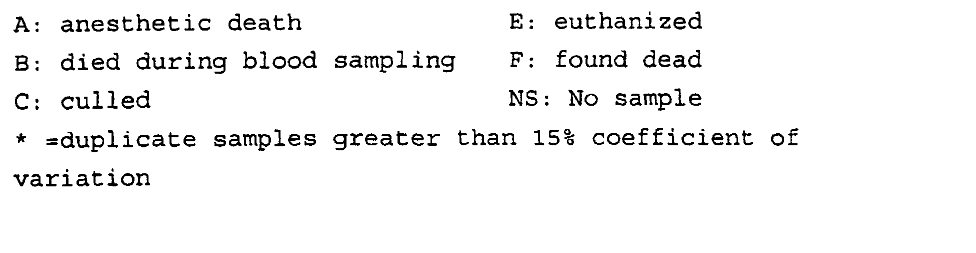

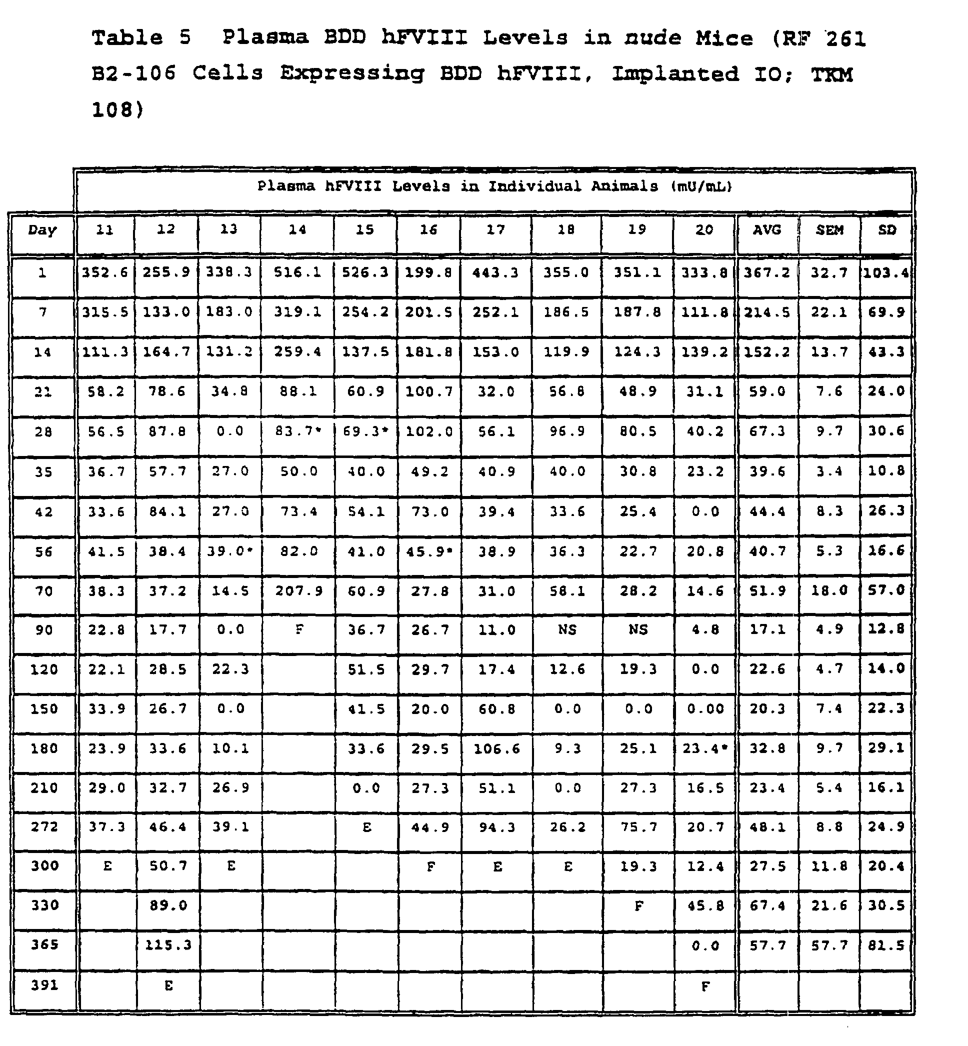

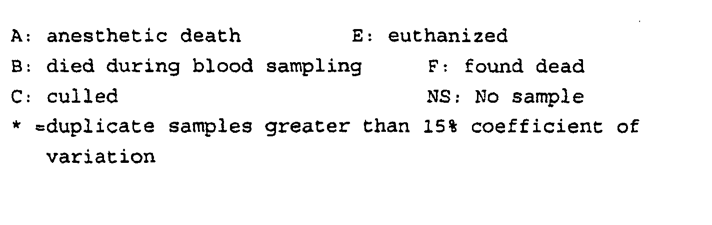

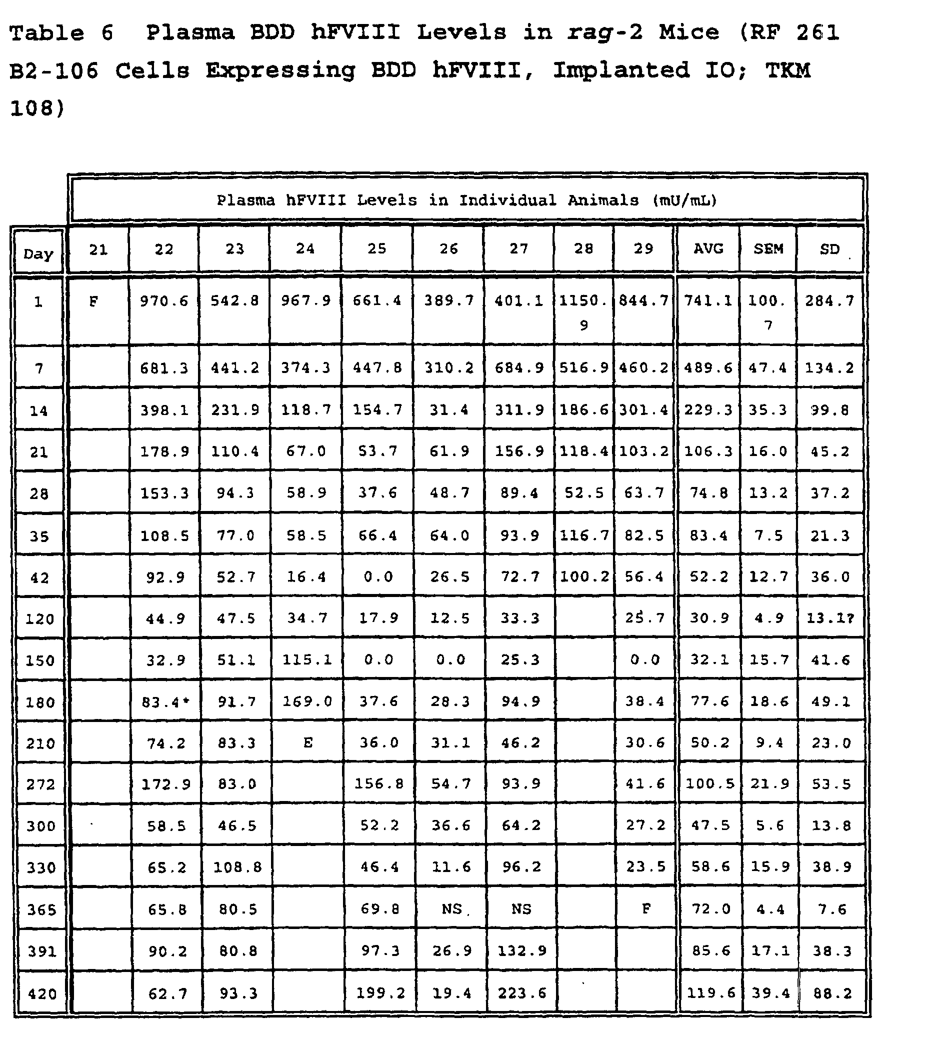

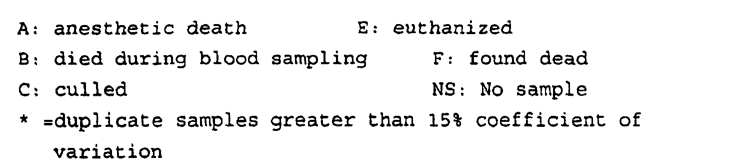

### Example 2: Comparison of the nude and rag-2 Mouse Models for Delivery of BDD hFVIII

hFVIII is known to be immunogenic, and many hemophiliacs are known to develop inhibitory antibodies to HFVIII.¹² Our primary mouse model, the athymic nude mouse, is known to be T-cell deficient. However, it does have a significant B-cell repertoire and may generate an immune response, primarily through IgM antibodies. The scid mouse is deficient in both T and B-cells, but the nature of the deficiency allows the occasional and spurious development of lymphocytes capable of producing antibody. In contrast, the *rag*-2 mouse is a knockout model in which the development of normal T and B-cells is precluded. The lack of T and B-cell development is due to an inability to conduct V(D)J rearrangement as a result of directed mutation of the recombination activating gene-2. Consequently, we chose to examine the *rag-*2 mouse as an alternative model system.

In the TKM 108 study described above, in which 5 x 10⁶ RF 261 B2-106 cells expressing BDD hFVIII were implanted IO in nude mice, a group of 10 female *rag*-2 mice was also implanted with 5 x 10⁶ RF 261 B2-106 cells, and long-term BDD hFVIII delivery in *rag*-2 mice was compared to the results obtained with nude mice. Five untreated female nude mice served as controls. The control group remained at or near undetectable levels for the duration of the experiment (Table 4). The data in Table 6 demonstrate that stable plasma BDD hFVIII levels in the range of 30-100 mU/mL were maintained for over 1 year in *rag*-2 mice and were clearly higher than the levels seen in nude mice.

### Example 3: Determination of the Optimal Cell Number for Intraomental Implantation

Three groups of 10 nude mice were implanted with either 5 x 10⁶, 2.5 X 10⁶, or 1.25 x 10⁶ RF 261 B2-106 cells per implant (TKM 111; see Tables 1 and 2 for details). The highest cell dosage, 5 x 10⁶ cells, was chosen as higher cell numbers proved difficult to implant. An additional 5 untreated nude mice served as controls. The results of this experiment are summarized in Table 7. In this experiment, some control animals showed spurious and inconsistent rises in hFVIII immunoreactivity, but the vast majority of samples remained below the detection limit of the assay. Through 419 days after implantation, the 5 x 10⁶ cell dosage group showed the highest plasma BDD hFVII levels at virtually all time points.

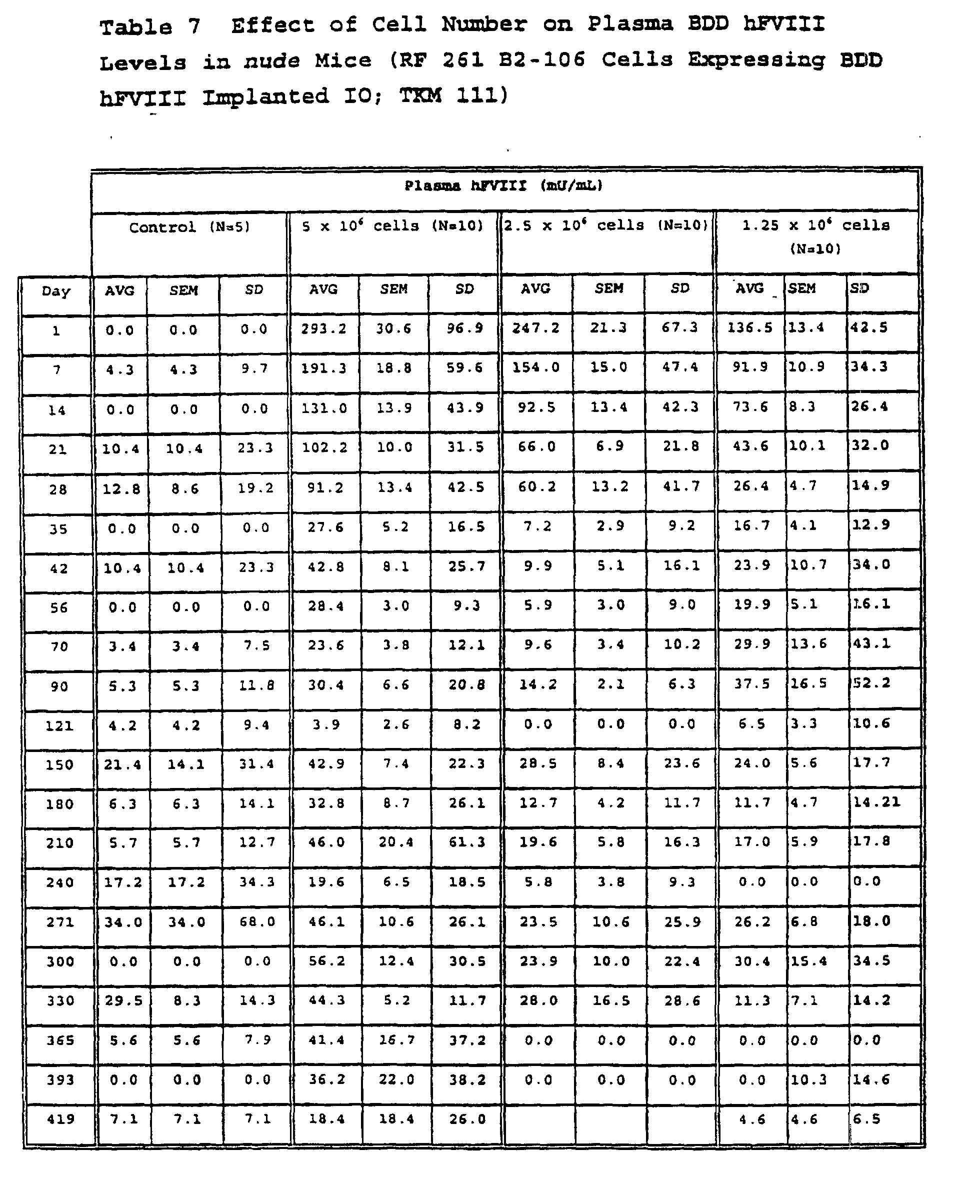

### Example 4: Comparison of Different Clonal Strains of Rabbit Fibroblasts Generated Using the BDD Expression Plasmid pXF8.61

Experiment TKM 132 was performed using the clonal rabbit fibroblast cell strain RF 288 B1-59, generated by transfecting rabbit fibroblasts with the BDD hFVIII expression plasmid pXF8.61 (the same plasmid used to generate the RF 261 B2-106 clonal strain used in experiments TKM 108 and 111 described above; see Table 2). 5 x 10⁶ cells were implanted IO in each of ten female nude mice. The data were compared to those obtained from nude mice in experiment TKM 108 (see Table 5). Plasma BDD hFVIII levels resulting from implantation of RF 288 B1-59 cells were sustained in the range of 10-50 mU/mL for over 250 days (Table 8) and were markedly higher than control values (TKM 126; Table 9). From Table 8, plasma BDD hFVIII levels were maintained over time at a level similar to that seen with IO implanted RF 261 B2-106 cells generated using the same transfecting plasmid.

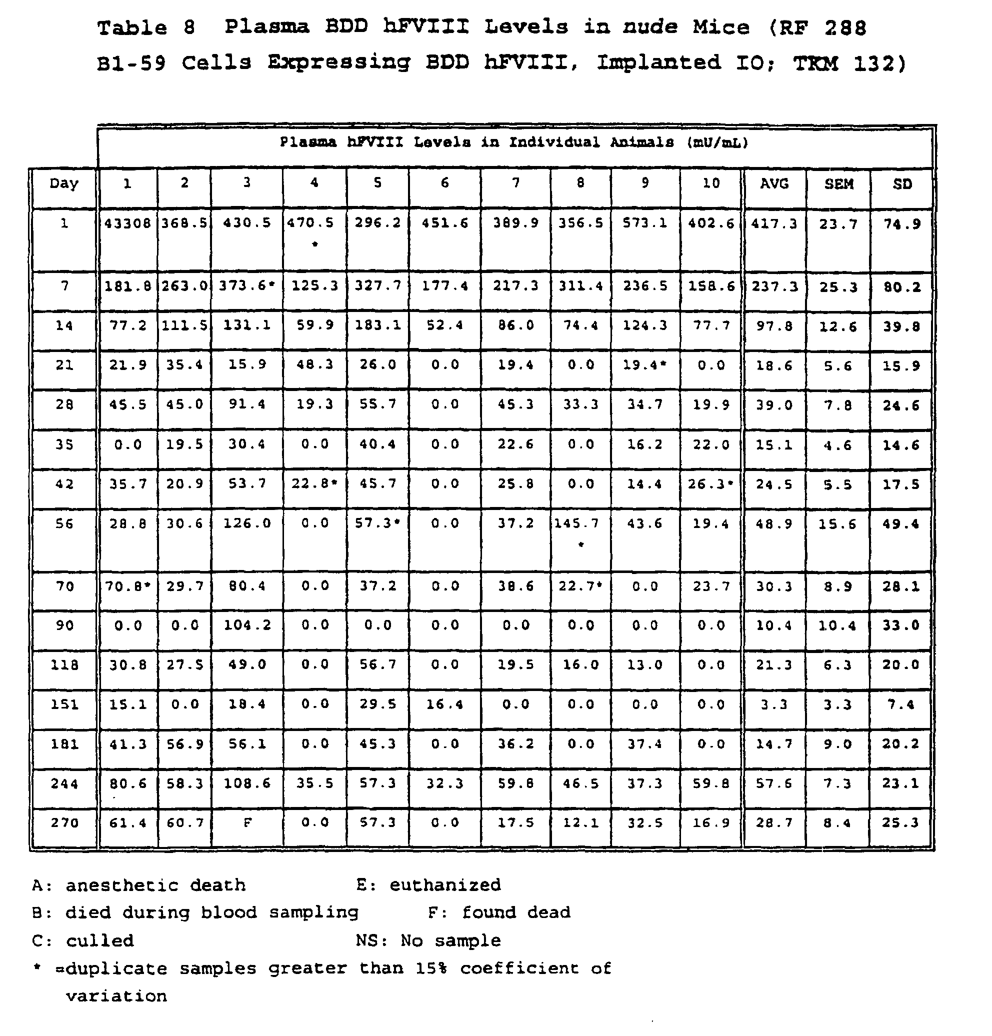

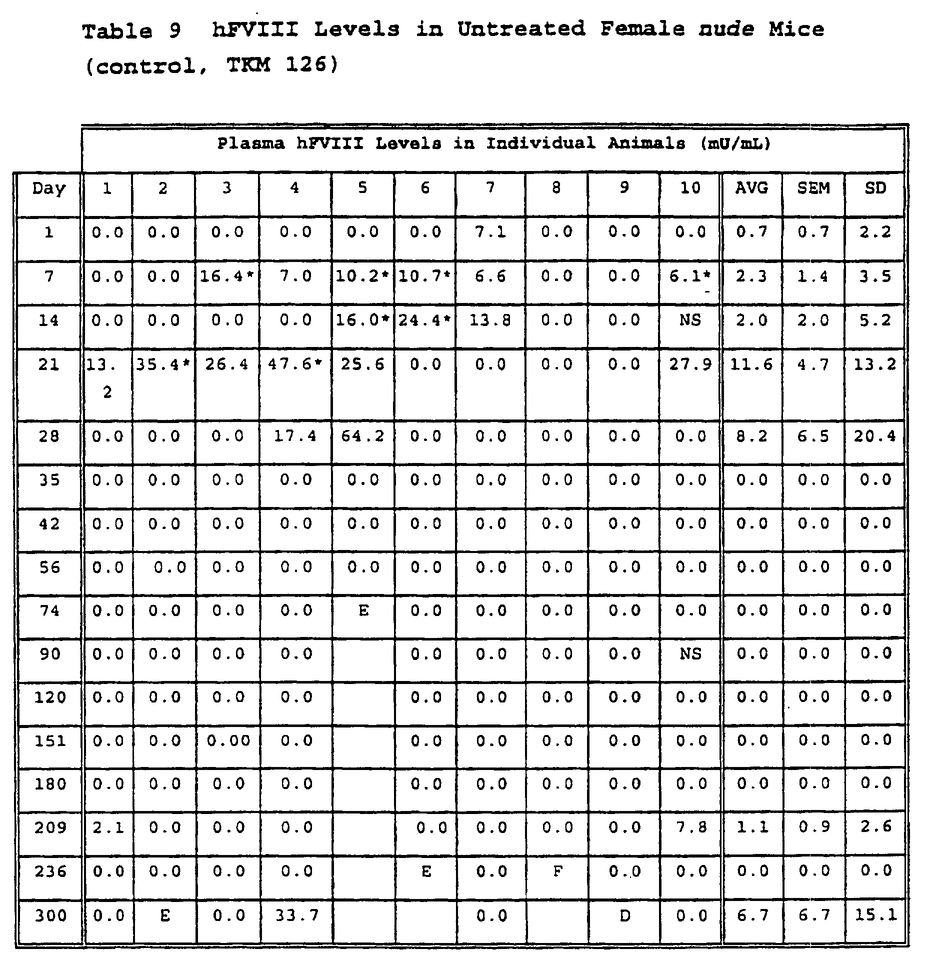

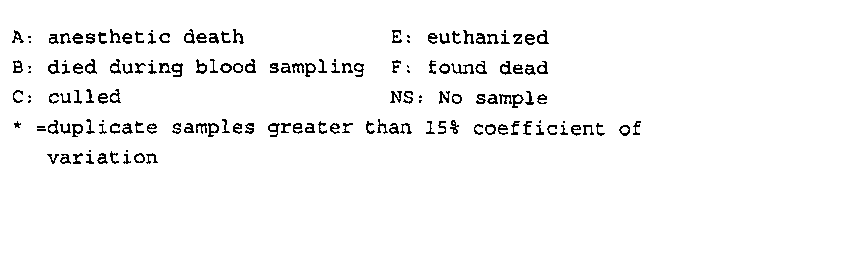

### Example 5: Comparison of Different Clonal Strains of Rabbit Fibroblasts Generated Using the BDD Expression Plasmid pXFB.186

Three clonal cell strains, PF 302 B2-382, RF 302 B2-383, and RF 302 B2-373, were generated using the BDD hFVIII expression construct pXF8.186. Each cell strain was implanted in a group of ten nude mice, and ten untreated nude mice served as controls. The results of these experiments are presented in Tables 10-13. All treatment groups showed elevated BDD hFVIII levels for the duration of the experiment (180 days).

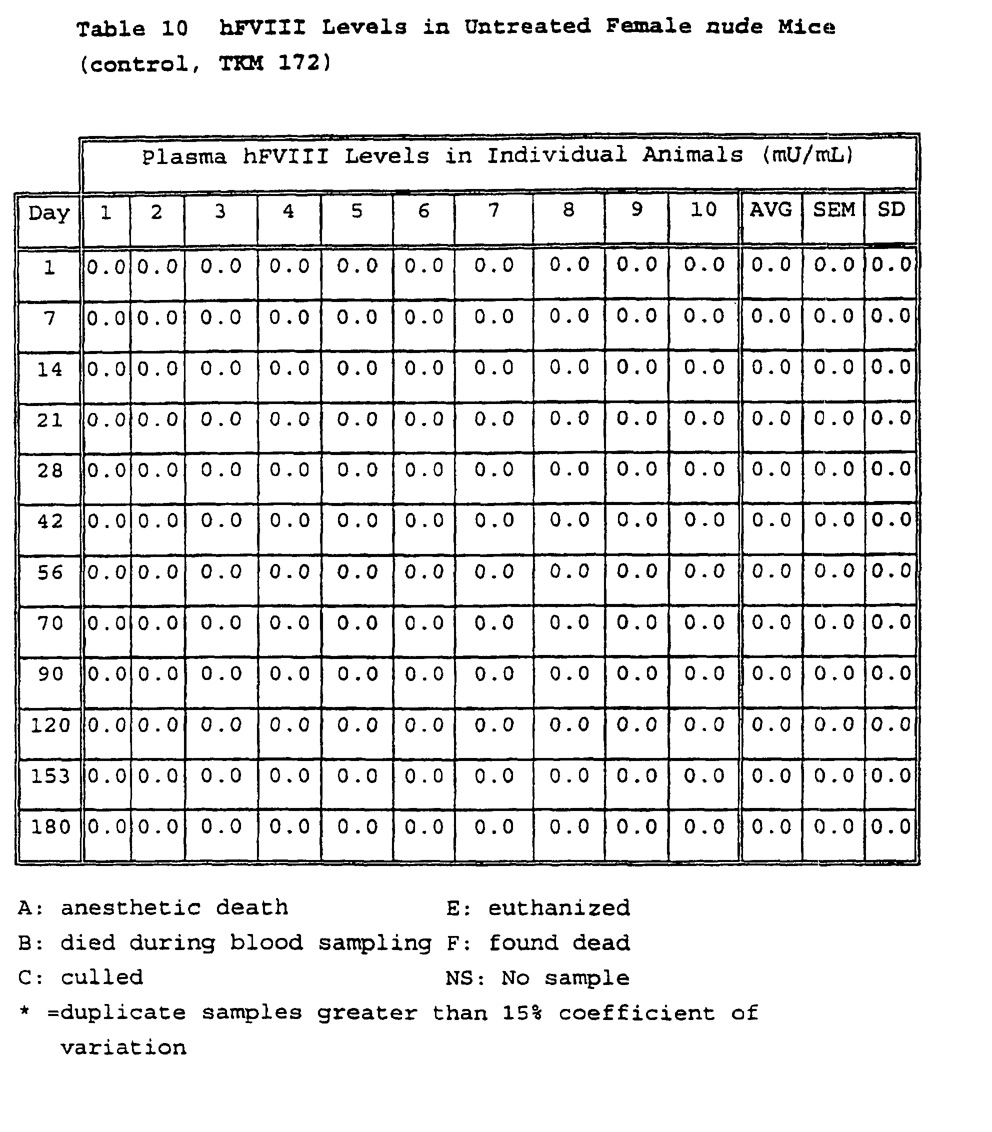

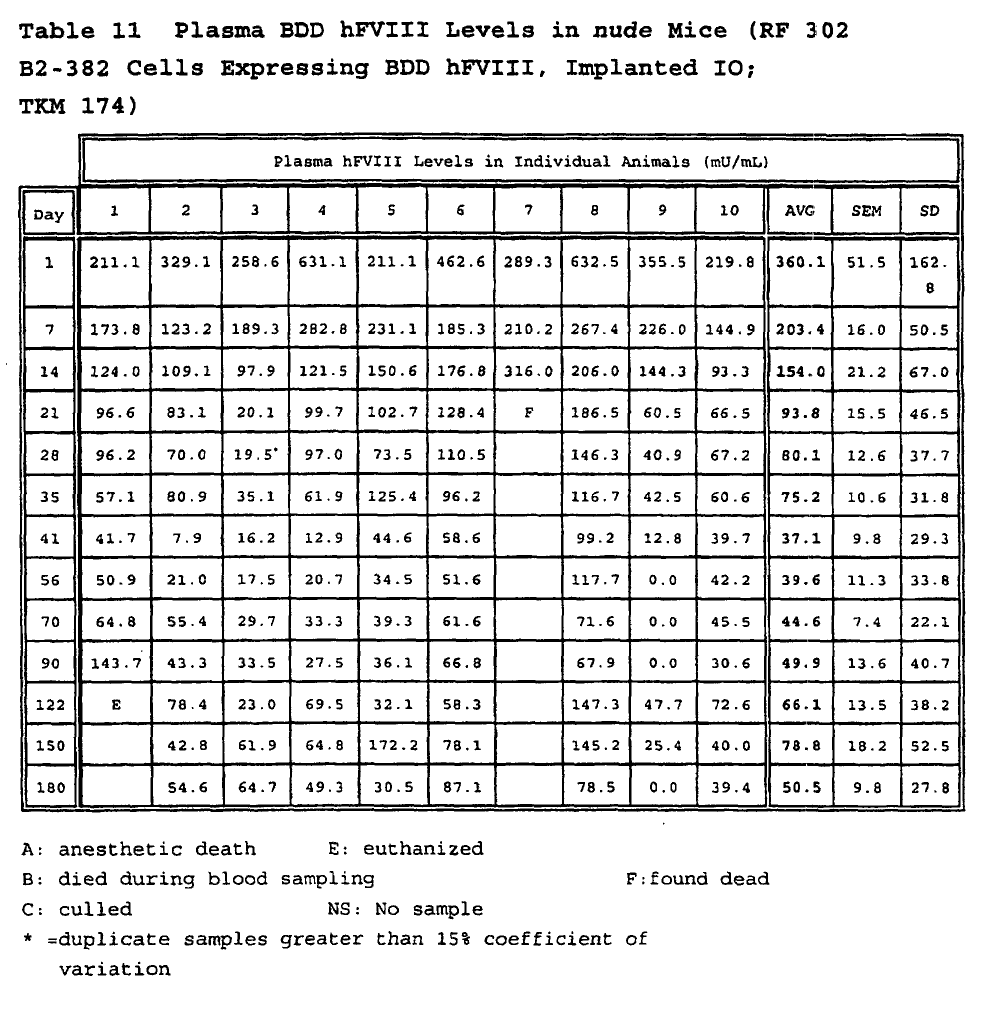

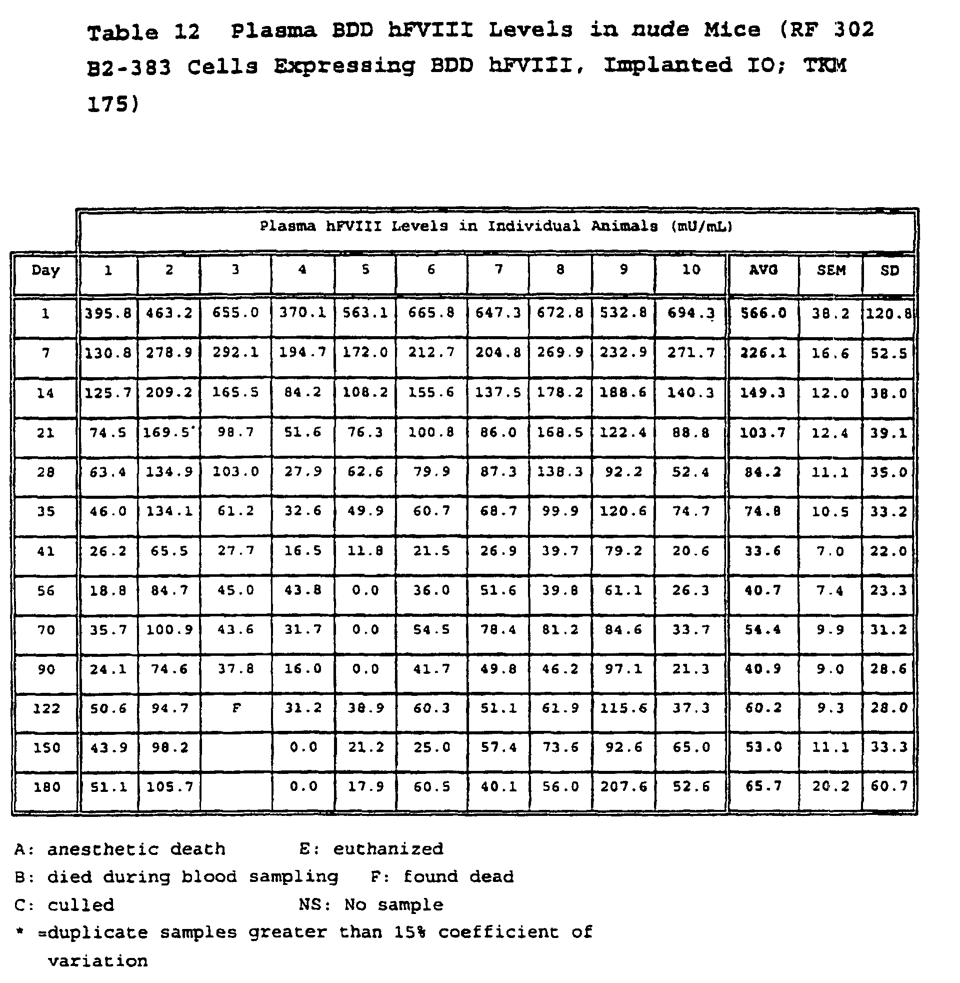

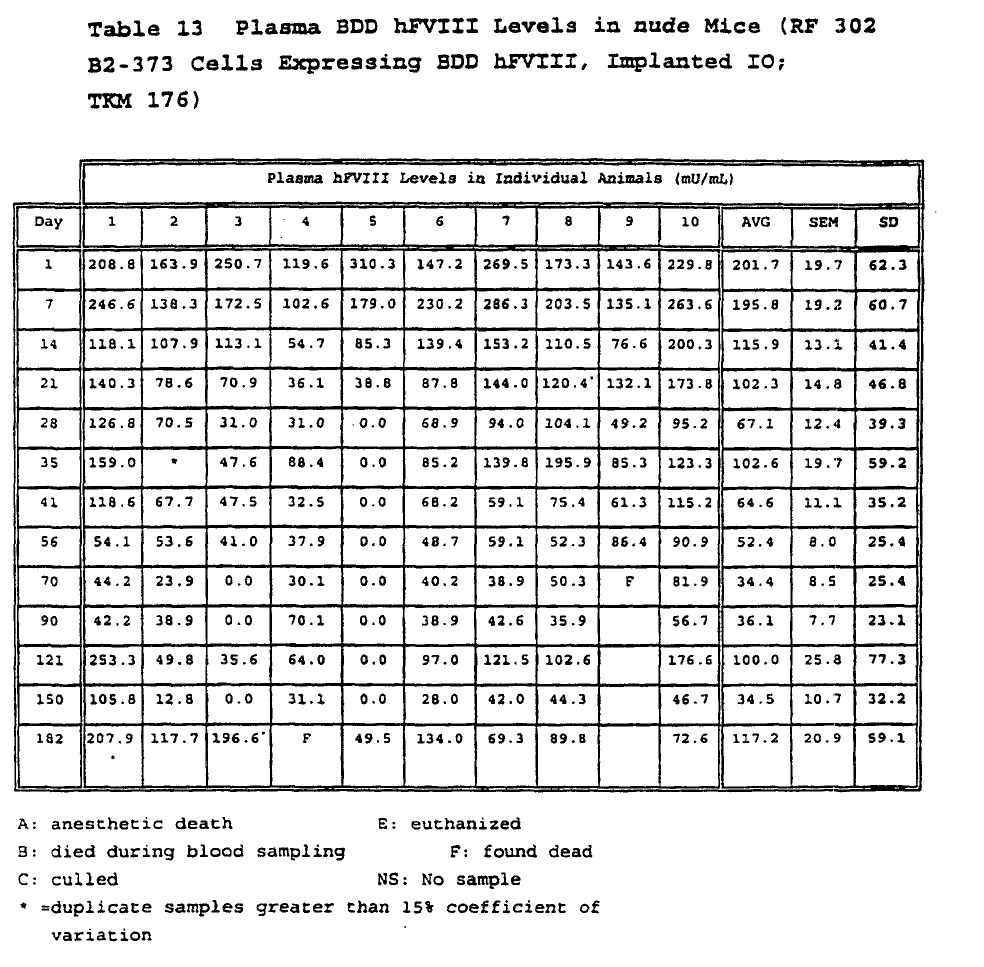

### Example 6: Implantation of Human Fibroblasts Producing BDD HFVIII into Male rag-2 Mice

IO implantation of human cells expressing another BDD HFVIII was tested in *rag-2* mice. Two clonal cell strains of human foreskin fibroblasts were generated using the BDD hFVIII expression plasmid pXFS.186. The clonal cell strains HP 701-18 and HF 701-27 were each implanted in groups of ten male *rag-2* mice (Tables 14 and 15). An additional ten male rag-2 mice served as controls (Table 16). In mice implanted with HF 701-27 cells, BDD hFVIII was detected in plasma for 120 days at levels of approximately 50 mU/mL.

Implants of clonal strains of transfected rabbit fibroblasts showed long-term *in vivo* function when implanted IO in immunocompromised mice, as judged by the detection of stable levels of BDD hFVIII in plasma for over 1 year. Cells implanted within the omentum gave vastly superior results as compared to cells implanted into other sites, including subcutaneous, intramuscular, subrenal capsular, mesenteric lymph node, and intraperitoneal implants.

The presence of BDD hFVIII in plasma has been confirmed by immunoprecipitation of plasma and Western blot analysis.

Implantation of 5 x 10⁶ transfected clonal cells per IO implant provided maximal BDD hFVIII plasma levels, as compared to implants of 2.5 x 10⁶ and 1.25 X 10⁶ cells.

*rag*-2 mice yielded higher plasma levels of hFVIII when compared to identically treated nude mice.

The results presented above demonstrate that stably transfected clonal skin fibroblasts expressing BDD hFVIII can lead to long-term (over 1 year) delivery of the clotting factor to the systemic circulation of immunocompromised mice. Plasma levels of 10-100 mU/ML were stably maintained, corresponding to 1-10% of the normal human level. As it is generally believed that increasing the level of hFVIII from <1% of normal levels, as seen in severe hemophilia A patients, to approximately 1-5% of normal levels, would dramatically improve the clotting phenotype of these patients, the above data suggest that administration of stably transfected clonal skin fibroblasts expressing BDD hFVIII can be used successfully for the treatment of hemophilia A.

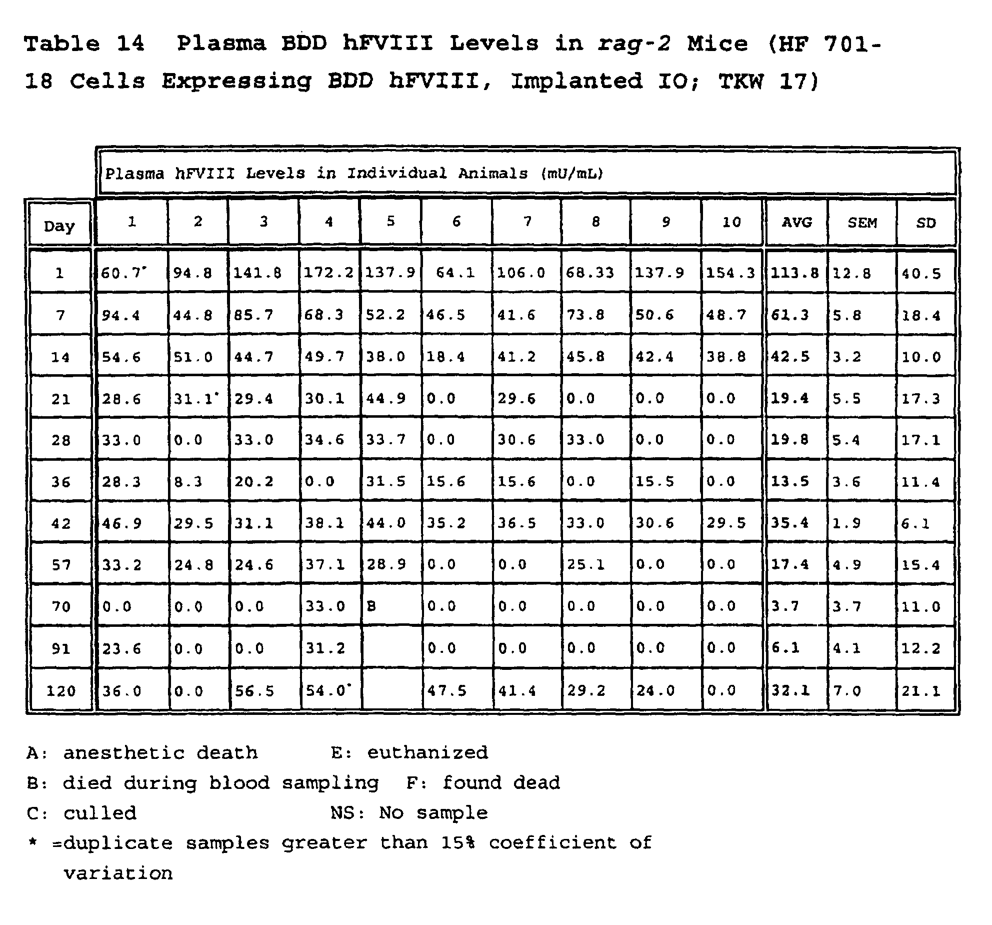

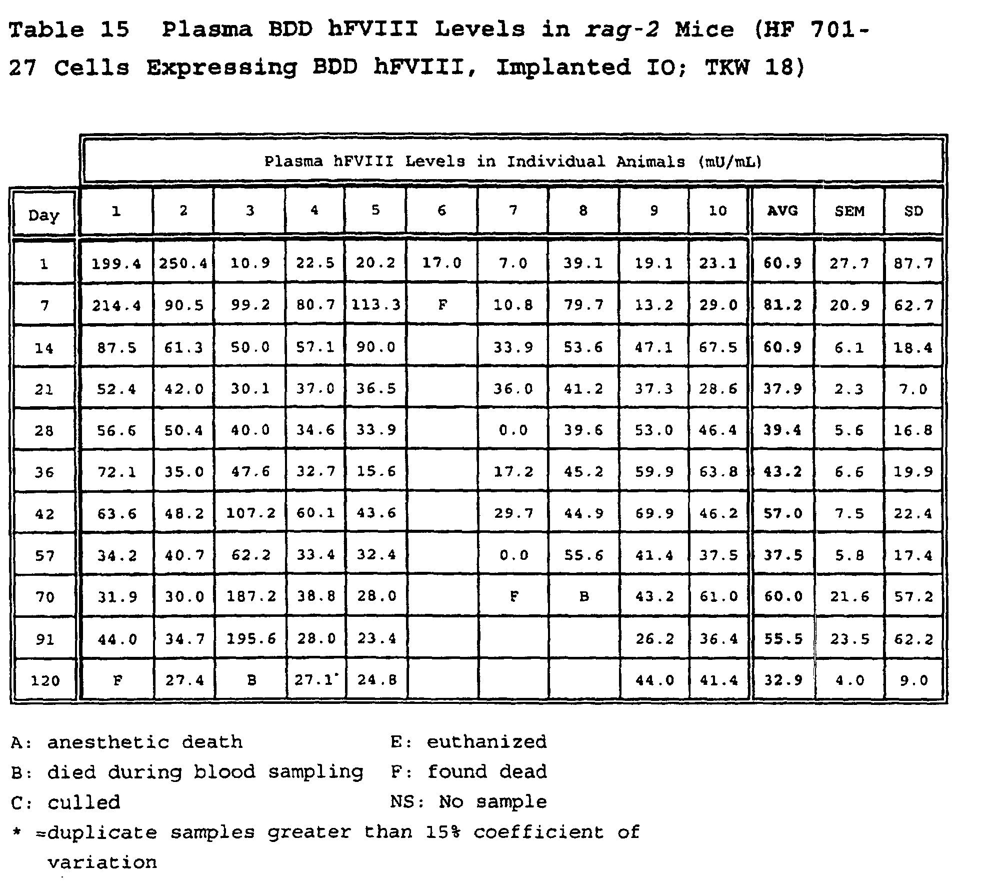

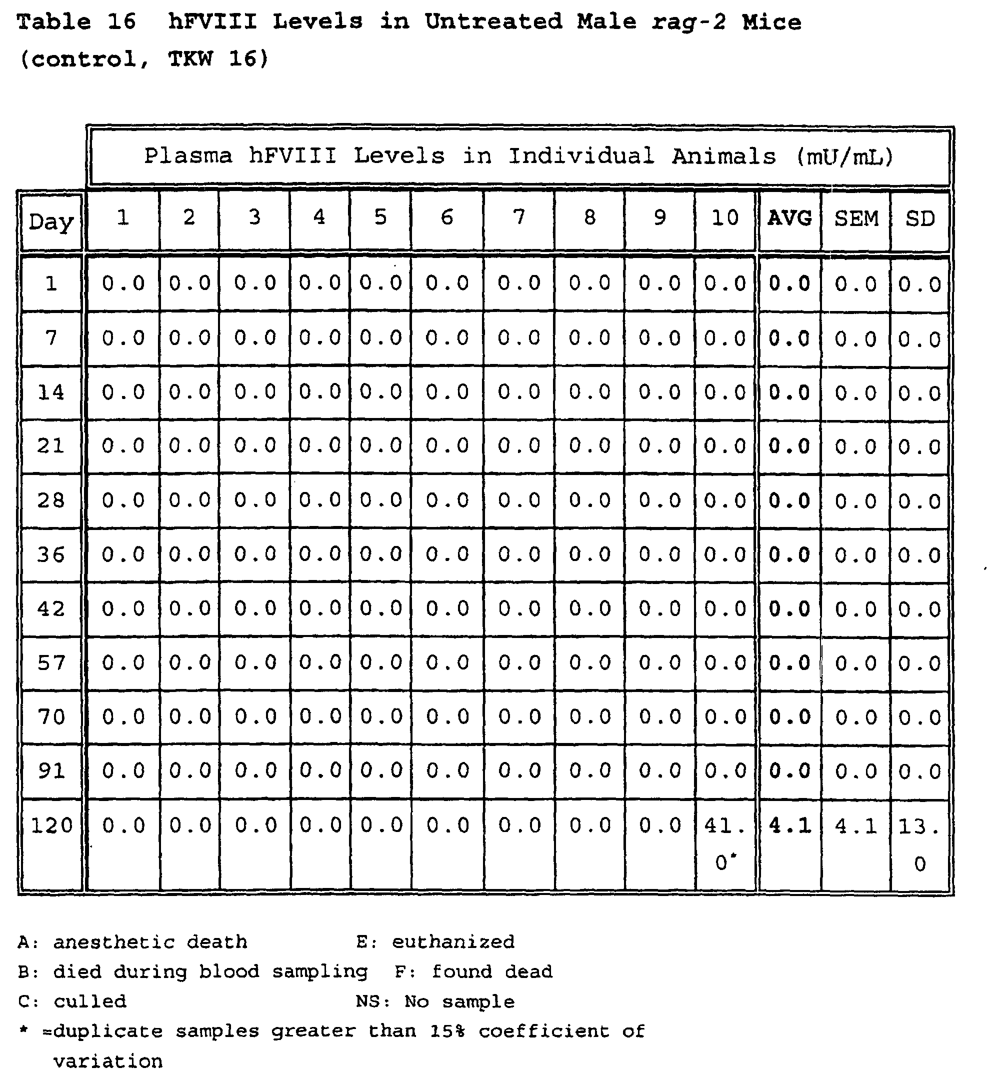

### Other embodiments

It is understood that while the invention has been described in conjunction with the detailed description thereof, the foregoing description is intended to illustrate and not limit the scope of the invention, which is defined by the scope of the appended claims.

## Claims

1. Use of a cell that is genetically engineered to secrete B domain-deleted Factor VIII for the manufacture of a medicament for the treatment of Hemophilia A in a mammal, wherein said cell is to be implanted into the omentum of the mammal.

2. The use of claim 1, wherein the mammal is a human.

3. The use of claim 1, wherein the cell is to be introduced into the omentum of the mammal by laparoscopy, surgical implantation, or direct injection.

4. The use of claim 1, wherein the cell is to be implanted into the omentum of the mammal in a collagen polymer.

5. The use of claim 1, wherein the promoter that controls expression of the B domain-deleted Factor VIII that is secreted by the cell is selected from group consisting of cytomegalovirus, collagen, and actin promoters.

6. The use of claim 1, wherein the implantation would result in the production of 10-100 mU/ml Factor VIII in the blood of the mammal.

7. The use of claim 1, wherein 1 x 10⁴ - 5 x 10¹⁰ cells that are genetically engineered to secrete B domain-deleted Factor VIII are to be implanted into the mammal.

8. The use of claim 1, wherein cells that are genetically engineered to secrete B domain-deleted Factor VIII are to be implanted into multiple sites of the omentum of the mammal.

9. Use of a cell that is genetically engineered to secrete B damain-deleted Factor VIII in the manufacture of a medicament for Factor VIII therapy, in a mammal in need of Factor VIII therapy, wherein said cell is to be introduced into the omentum of the mammal.

10. The use of claim 9, wherein the mammal is a human.

11. The use of claim 9, wherein the cell is to be introduced into the omentum of the mammal by laparoscopy, surgical implantation, or direct injection.

12. The use of claim 9, wherein the cell is to be implanted into the omentum of the mammal in a collagen polymer.

13. The use of claim 9, wherein the promoter that controls expression of the B domain-deleted Factor VIII that is secreted by the cell is selected from group consisting of cytomegalovirus, collagen, and actin promoters.

14. The use of claim 9, wherein the implantation would result in the production of 10-100 mU/ml Factor VIII in the blood of the mammal.

15. The use of claim 9, wherein 1 x 10⁴ - 5 x 10¹⁰ cells that are genetically engineered to secrete B domain-deleted Factor VIII are to be implanted into the mammal.

16. The use of claim 9, wherein cells that are genetically engineered to secrete B domain-deleted Factor VIII are to be implanted into multiple sites of the omentum of the mammal.

## Patentansprüche

1. Verwendung einer Zelle, die genetisch verändert ist um einen B-Domänen-deletierten Faktor VIII zu sekretieren, zur Herstellung eines Arzneimittels für die Behandlung von Hämophilie A in einem Säugetier, wobei die Zelle in das Omentum des Säugetiers implantiert wird.

2. Verwendung nach Anspruch 1, wobei das Säugetier ein Mensch ist.

3. Verwendung nach Anspruch 1, wobei die Zelle in das Omentum des Säugetiers durch Laparoskopie, operative Implantation oder direkte Injektion eingeführt wird.

4. Verwendung nach Anspruch 1, wobei die Zelle in das Omentum des Säugetiers in ein Kollagen-Polymer implantiert wird.

5. Verwendung nach Anspruch 1, wobei der Promotor, der die Expression des von der Zelle sekretieren B-Domänen-deletierten Faktors VIII kontrolliert, ausgewählt ist aus der Gruppe bestehend aus Cytomegalovirus-, Kollagen- und Aktin-Promotoren.

6. Verwendung nach Anspruch 1, wobei die Implantation in die Erzeugung von 10-100 mE/ml Faktor VIII im Blut des Säugetiers resultieren würde.

7. Verwendung nach Anspruch 1, wobei 1 x 10⁴ - 5 x 10¹⁰ Zellen, die genetisch verändert sind um B-Domänen-deletierten Faktor VIII zu sekretieren, in das Säugetier implantiert werden.

8. Verwendung nach Anspruch 1, wobei die Zellen, die genetisch verändert sind um B-Domänen-deletierten Faktor VIII zu sekretieren, an mehreren Stellen des Omentums von dem Säugetier implantiert werden.

9. Verwendung einer Zelle, die genetisch verändert wurde um einen B-Domänen-deletierten Faktor VIII zu sekretieren, zur Herstellung eines Arzneimittels für eine Faktor VIII-Therapie in einem Säugetier, das eine Faktor VIII-Therapie benötigt, wobei die Zelle in das Omentum des Säugetiers eingeführt wird.

10. Verwendung nach Anspruch 9, wobei das Säugetier ein Mensch ist.

11. Verwendung nach Anspruch 9, wobei die Zelle in das Omentum des Säugetiers durch Laparoskopie, operative Implantation oder direkte Injektion eingeführt wird.

12. Verwendung nach Anspruch 9, wobei die Zelle in das Omentum des Säugetiers in ein Kollagen-Polymer eingeführt wird.

13. Verwendung nach Anspruch 9, wobei der Promotor, der die Expression des von der Zelle sekretierten B-Domänen-deletierten Faktors VIII kontrolliert, ausgewählt ist aus der Gruppe bestehend aus Cytomegalovirus-, Kollagen- und Aktin-Promotoren.

14. Verwendung nach Anspruch 9, wobei die Implantation in die Erzeugung von 10-100 mE/ml Faktor VIII im Blut des Säugetiers resultieren würde.

15. Verwendung nach Anspruch 9, wobei 1 x 10⁴ - 5 x 10¹⁰ Zellen, die genetisch verändert sind um B-Domänen-deletierten Faktor VIII zu sekretieren, in das Säugetier implantiert werden.

16. Verwendung nach Anspruch 9, wobei die Zellen, die genetisch verändert sind um B-Domänen-deletierten Faktor VIII zu sekretieren, an mehreren Stellen des Omentums von dem Säugetier implantiert werden.

## Revendications

1. Utilisation d'une cellule qui est traitée par génie génétique pour sécréter du facteur VIII à domaine B délété, pour la préparation d'un médicament pour le traitement de l'hémophilie A chez un mammifère, dans laquelle ladite cellule est destinée à être implantée dans l'omentum du mammifère.

2. Utilisation selon la revendication 1, dans laquelle le mammifère est un humain.

3. Utilisation selon la revendication 1, dans laquelle la cellule est destinée à être introduite dans l'omentum du mammifère par laparoscopie, implantation chirurgicale, ou injection directe.

4. Utilisation selon la revendication 1, dans laquelle la cellule est destinée à être implantée dans l'omentum du mammifère dans un polymère de collagène.

5. Utilisation selon la revendication 1, dans laquelle le promoteur qui commande l'expression du facteur VIII à domaine B délété qui est sécrété par la cellule est choisi dans le groupe consistant en les promoteurs de type cytomégalovirus, collagène, et actine.

6. Utilisation selon la revendication 1, dans laquelle l'implantation aurait pour résultat la production de 10-100 mU/ml de facteur VIII dans le sang du mammifère.

7. Utilisation selon la revendication 1, dans laquelle 1 x 10⁴ - 5 x 10¹⁰ cellules qui sont traitées par génie génétique pour sécréter le facteur VIII à domaine B délété sont destinées à être implantées dans le mammifère.

8. Utilisation selon la revendication 1, dans laquelle les cellules qui sont traitées par génie génétique pour sécréter le facteur VIII à domaine B délété sont destinées à être implantées dans des sites multiples de l'omentum du mammifère.

9. Utilisation d'une cellule qui est traitée par génie génétique pour sécréter le facteur VIII à domaine B délété dans la préparation d'un médicament pour la thérapie du facteur VIII, chez un mammifère ayant besoin d'une thérapie au facteur VIII, tandis que ladite cellule est destinée à être introduite dans l'omentum du mammifère.

10. Utilisation selon la revendication 9, dans laquelle le mammifère est un humain.

11. Utilisation selon la revendication 9, dans laquelle la cellule est destinée à être introduite dans l'omentum du mammifère par laparoscopie, implantation chirurgicale, ou injection directe.

12. Utilisation selon la revendication 9, dans laquelle la cellule est destinée à être implantée dans l'omentum du mammifère dans un polymère de collagène.

13. Utilisation selon la revendication 9, dans laquelle le promoteur qui commande l'expression du facteur VIII à domaine B délété qui est sécrété par la cellule est choisi dans le groupe consistant en les promoteurs de type cytomégalovirus, collagène, et actine.

14. Utilisation selon la revendication 9, dans laquelle l'implantation aurait pour résultat la production de 10-100 mU/ml de facteur VIII dans le sang du mammifère.

15. Utilisation selon la revendication 9, dans laquelle 1 x 10⁴ - 5 x 10¹⁰ cellules qui sont traitées par génie génétique pour sécréter le facteur VIII à domaine B délété sont destinées à être implantées dans le mammifère.

16. Utilisation selon la revendication 9, dans laquelle les cellules qui sont traitées par génie génétique pour sécréter le facteur VIII à domaine B délété sont destinées à être implantées dans des sites multiples de l'omentum du mammifère.
